# EUROPEAN PATENT SPECIFICATION

(11) **EP 4 627 340 B1**
(45) Date of publication and mention of the grant of the patent: **14.10.2026**
(21) Application number: 23828646.2
(22) Date of filing: 27.11.2023
(51) Int. Cl.: G01N 30/88

(54) **METHOD OF PROCESSING ETANERCEPT**
VERFAHREN ZUR AUSWERTUNG VON ETANERCEPT
MÉTHODE DE TRAITEMENT DE ETANERCEPT

(30) Priority: 28.11.2022 US 202263428233 P
(43) Date of publication of application: 08.10.2025
(73) Proprietor: Amgen Inc., Thousand Oaks, CA 91320 (US)
(72) Inventor: REN, Da, Thousand Oaks, CA 91320 (US); SONG, Ting, Thousand Oaks, CA 91320 (US); APOSTOL, Izydor, Thousand Oaks, CA 91320 (US)
(74) Representative: J A Kemp LLP
(86) International application number: PCT/US2023/081113
(87) International publication number: WO 2024/118481

(56) References cited:
- JP-A- 2020 521 460
- QINGQIAO TAN ET AL: "Characterization and comparison of commercially available TNF receptor 2-Fc fusion protein products", MABS, vol. 4, no. 6, 1 November 2012 (2012-11-01), US, pages 761 - 774, XP055285200, ISSN: 1942-0862, DOI: 10.4161/mabs.22276
- LI-JUAN HUANG ET AL: "Complete mapping of disulfide linkages for etanercept products by multi-enzyme digestion coupled with LC-MS/MS using multi-fragmentations including CID and ETD", JOURNAL OF FOOD AND DRUG ANALYSIS, vol. 27, no. 2, 1 January 2019 (2019-01-01), pages 531 - 541, XP055703629, DOI: 10.1016/j.jfda.2018.11.007
- LARGY ERIC ET AL: "Orthogonal liquid chromatography-mass spectrometry methods for the comprehensive characterization of therapeutic glycoproteins, from released glycans to intact protein level", JOURNAL OF CHROMATOGRAPHY A, vol. 1498, 1 May 2017 (2017-05-01), AMSTERDAM, NL, pages 128 - 146, XP093138211, ISSN: 0021-9673, Retrieved from the Internet <URL:https://www.sciencedirect.com/science/article/pii/S002196731730331X/pdfft?md5=7f7b709e64ad34e655db11d7403d3c91&pid=1-s2.0-S002196731730331X-main.pdf> [retrieved on 20240306], DOI: 10.1016/j.chroma.2017.02.072
- TOHRU IKEGAMI: "Hydrophilic interaction chromatography for the analysis of biopharmaceutical drugs and therapeutic peptides: A review based on the separation characteristics of the hydrophilic interaction chromatography phases", JOURNAL OF SEPARATION SCIENCE, vol. 42, no. 1, 1 January 2019 (2019-01-01), DE, pages 130 - 213, XP055713038, ISSN: 1615-9306, DOI: 10.1002/jssc.201801074
- SRIKANTH JANDHYAM ET AL: "Targeted, Site-specific quantitation of N- and O-glycopeptides using18O-labeling and product ion based mass spectrometry", GLYCOCONJUGATE JOURNAL, CHAPMAN & HALL, BOSTON, vol. 34, no. 1, 6 October 2016 (2016-10-06), pages 95 - 105, XP036138167, ISSN: 0282-0080, [retrieved on 20161006], DOI: 10.1007/S10719-016-9733-8

## Description

### FIELD

The disclosure relates to a method of processing etanercept.

### BACKGROUND

Etanercept is a highly glycosylated molecule, containing both N- and O-linked oligosaccharides which potentially influence the activity and immunogenicity of the therapeutic. Glycosylation, including sialylation, is a common protein therapeutic attribute characterized in stability studies. Processing and characterizing glycosylation species of etanercept is complicated due to the molecule's complexity and the high acidity associated with sialic acid. Current methods used to characterize etanercept glycosylation release all of the glycans from the protein backbone or remove the sialic acid and N-glycans, which cause the loss of glycosylation site information. There remains a need in the art for a method of processing etanercept which enables efficient characterization of N- and O-glycosylation.

Tan et al., Mabs. 4(6):761-774 (2012) describes the characterization and comparison of commercially available TNF receptor 2-Fc fusion protein products. Huang et al., J Food Drug Anal. 27(2):531-541 (2019) describes complete mapping of disulfide linkages for etanercept products by multi-enzyme digestion coupled with LC-MS/MS using multi-fragmentations including CID and ETD. JP 2020 521460A (Genovis AB) describes endoprostheses, mutants thereof that have binding activity but lack or have reduced hydrolytic activity, and their use in methods for studying and isolating O-linked glycoproteins. Largy et al., J Chromatogr A. 1498:128-146 (2017) describes orthogonal liquid chromatography-mass spectrometry methods for the comprehensive characterization of therapeutic glycoproteins, from released glycans to intact protein level. Ikegami, J Sep Sci. 42(1):130-213 (2019) describes hydrophilic interaction chromatography for the analysis of biopharmaceutical drugs and therapeutic peptides. Srikanth et al., Glycoconj J. 34(1):95-105 (2017) describes targeted, site-specific quantitation of N- and O-glycopeptides using ¹⁸O-labeling and product ion based mass spectrometry.

### SUMMARY

The invention is defined by the appended claims. Any part of the description which does not fall under the scope of the appended claims is for illustrative purposes only. In particular, any subject-matter referred to as "disclosure" which is not falling under the scope of the appended claims does not form part of the invention.

The present disclosure includes a computer-readable nucleotide/amino acid sequence listing (SEQ ID NO 1 and SEQ ID NO 2).

The invention provides a method of processing etanercept. The method comprises (a) digesting etanercept with trypsin and Arg-C at an etanercept:trypsin:Arg-C ratio of 100:[1-100]:[1-100] to produce a pool of peptide fragments; (b) applying the peptide fragments to a reverse-phase chromatography column; and (c) eluting the peptide fragments using a gradient consisting of variable amounts of a mobile phase A comprising water and 0.02%-0.5% formic acid or 0.01%-0.5% trifluoroacetic acid (TFA) and a mobile phase B comprising acetonitrile and 0.02%-0.5% formic acid or 0.01%-0.5% TFA at a flow rate of 1 µL/min to 800 µl/min over a time period of at least 15 minutes. Optionally, (i) step (a) comprises digesting etanercept with trypsin and Arg-C at an etanercept:trypsin:Arg-C ratio of 20:1:4; (ii) step (a) is performed for about 1-12 hours (e.g., 2 hours); (iii) step (a) is performed at a temperature of about 37° C ± 5° C; and/or (iv) step (a) is performed in a buffer comprising Tris-HCl, CaCl₂, ethylenediaminetetraacetic acid (EDTA), and dithiothreitol (DTT) (any combination of (i)-(iv) is contemplated). In various aspects, the reverse-phase column is a C18 chromatography column and/or step (b) comprises injecting about 1 µg to about 25 µg of peptide fragments to the column. In various aspects, (i) step (c) comprises eluting the peptide fragments using a gradient consisting of variable amounts of a mobile phase A comprising water and about 0.1% formic acid and a mobile phase B comprising acetonitrile and about 0.1% formic acid; (ii) step (c) is performed using a flow rate of about 200 µL/min to about 750 µl/min (e.g., about 200 µL/min) over a time period of at least about 120 minutes; and/or (iii) step (c) is performed at a column temperature of about 50° C ± 5° C (any combination of (i)-(iii) is contemplated). Optionally, the method also comprises (d) analyzing the eluted peptide fragments by spectrometric analysis, such as LC-MS.

It should be understood that, while various embodiments in the specification are presented using "comprising" language, under various circumstances, a related embodiment may also be described using "consisting of" or "consisting essentially of' language. The disclosure contemplates embodiments described as "comprising" a feature to include embodiments which "consist of" or "consist essentially of' the feature. The term "a" or "an" refers to one or more. As such, the terms "a" (or "an"), "one or more," and "at least one" can be used interchangeably herein. The term "or" should be understood to encompass items in the alternative or together, unless context unambiguously requires otherwise.

It should also be understood that when describing a range of values, the disclosure contemplates individual values found within the range. For example, "about 0.02%-0.5% formic acid," could be, but is not limited to, about 0.02%, about 0.025%, about 0.05%, about 0.75%, about 0.1%, about 0.15%, about 0.25%, etc., and any value in between such values. In any of the ranges described herein, the endpoints of the range are included in the range. However, the description also contemplates the same ranges in which the lower and/or the higher endpoint is excluded. When the term "about" is used, it means the recited number plus or minus 5%, 10%, or more of that recited number. The actual variation intended is determinable from the context.

### BRIEF DESCRIPTION OF THE DRAWINGS

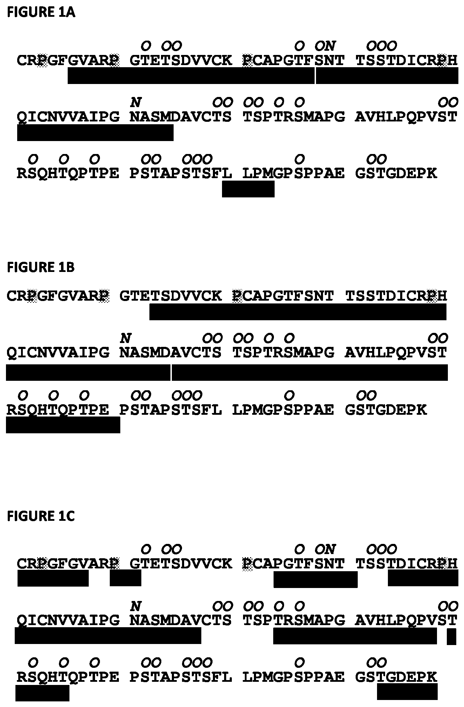

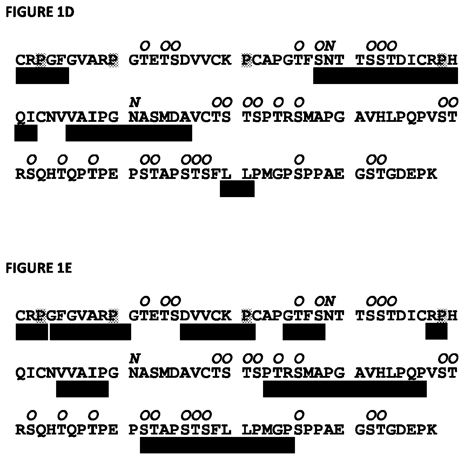

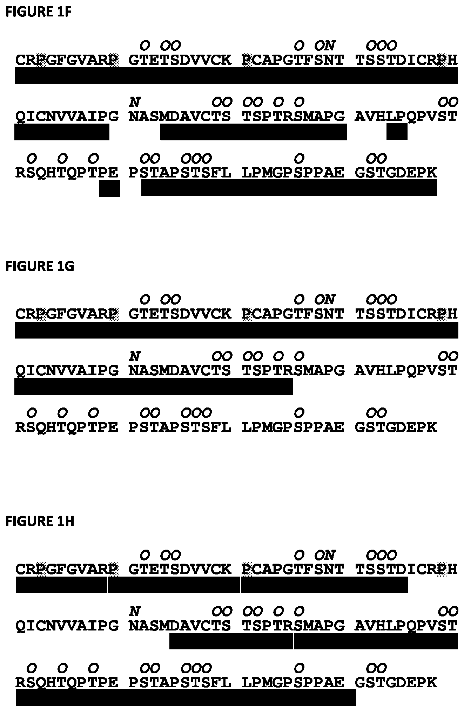

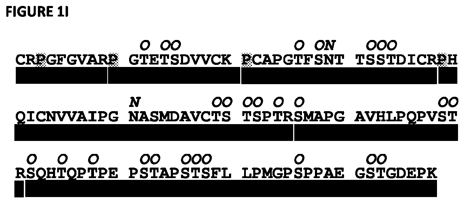

Figures 1A-1I are illustrations representing LC-MS detectable digest products achieved using different enzyme digest preparations. Amino acids 121-238 of etanercept are depicted for illustration purposes (SEQ ID NO: 2). Figure 1A is a peptide sequence coverage map achieved using chymotrypsin digestion. Figure 1B is a peptide sequence coverage map achieved using Glu-C digestion. Figure 1C is a peptide sequence coverage map achieved using Human Neutrophil Elastase (HNE) digestion. Figure 1D is a peptide sequence coverage map achieved using pepsin digestion. Figure 1E is a peptide sequence coverage map achieved using ProAlanase digestion. Figure 1F is a peptide sequence coverage map achieved using pronase digestion. Figure 1G is a peptide sequence coverage map achieved using trypsin digestion. Figure 1H is a peptide sequence coverage map achieved using trypsin/chymotrypsin digestion. Figure 1I is a peptide sequence coverage map achieved using trypsin/Arg-C digestion. Notations on the figures represent the following: O above an amino acid denotes O-glycosylation; N above an amino acid denotes N-glycosylation; shaded P denotes a proline after an arginine or lysine; solid bar underneath peptide sequences denote peptides detected by LC-MS; and a blank space underneath peptide sequences denote peptides not detected by LC-MS. Methods using a digest step comprising exposure of etanercept to trypsin and Arg-C as described herein allowed more accurate detection of etanercept (i.e., more fragments covering the length of the etanercept protein were detectable using LC-MS).

### DETAILED DESCRIPTION

Etanercept (Enbrel^{®}, Immunex Corporation) is a dimeric fusion polypeptide consisting of the extracellular ligand-binding portion of the human 75 kilodalton (p75) tumor necrosis factor receptor (TNFR) linked to the Fc portion of human IgG1 (see, e.g., U.S. Patent No. 9,518,111). The Fc component of etanercept contains the constant heavy 2 (CH2) domain, the constant heavy 3 (CH3) domain and hinge region, but not the constant heavy 1 (CH1) domain of human IgG1. It consists of 934 amino acids and has an apparent molecular weight of approximately 150 kilodaltons (Physicians Desk Reference, 2002, Medical Economics Company Inc.). Etanercept has a complicated N-linked and O-linked glycosylation pattern in the TNFR region as well as N-glycosylation within the CH₂ region. It has been estimated that glycosylation represents about 30% of etanercept's molecular weight. It was previously believed that characterization of glycosylated species of etanercept via liquid chromatography-mass spectrometry (LC-MS) required removal of sialic acid residues. Surprising, using the method described herein, the sialic acid content of etanercept can be preserved and quantified. Further, previous methods of digestion generated few, large peptide fragments, which complicates peptide mapping data analysis due to the complexity of the fragments. The method of the disclosure, in addition to preserving glycosylation information, allows generation of a pool of shorter peptide fragments suitable for liquid chromatography separation and mass spectroscopy analysis which increases the confidence of peptide identification, characterization, and quantitation.

The disclosure provides a method of processing etanercept. The method comprises (a) digesting etanercept with trypsin and Arg-C at an etanercept:trypsin:Arg-C ratio of about 100:[1-100]:[1-100] to produce a pool of peptide fragments; (b) applying the peptide fragments to a reverse-phase chromatography column; and (c) eluting the peptide fragments using a gradient comprising variable amounts of a mobile phase A comprising water and about 0.02%-0.5% formic acid or about 0.01%-0.5% trifluoroacetic acid (TFA) and a mobile phase B comprising acetonitrile and about 0.02%-0.5% formic acid or about 0.01%-0.5% TFA at a flow rate of about 1 µL/min to about 800 µl/min over a time period of at least about 15 minutes. In various aspects, step (a) comprises digesting etanercept with trypsin and Arg-C and Lys-C at an etanercept:trypsin:Arg-C:Lys-C ratio of about 100:[1-100]:[1-100]:[1-100] to produce a pool of peptide fragments. In various aspects of the disclosure, the method does not comprise use of sialidase.

In the method of the disclosure, etanercept is digested with trypsin and Arg-C. Arg-C is an endopeptidase (clostripain) that cleaves at the C-terminus of arginine residues as well as lysine residues. Trypsin is a serine protease that cleaves at the carboxyl side of the amino acids lysine and arginine. Trypsin and Arg-C are not typically combined or used together because of the cleavage patterns of the two enzymes, which produce redundant peptide fragments. Surprisingly, use of both enzymes results in a digest suitable for accurately characterizing glycosylated species of etanercept via LC-MS. The amounts of etanercept, trypsin, and Arg-C in the digest result in a ratio of about 100 (etanercept) to about 1 to about 100 (trypsin) to about 1 to about 100 (Arg-C). In various aspects, the ratio of etanercept to trypsin is about 100 (etanercept) to about 1-10 (trypsin), such as about 100 (etanercept) to about 1-9 (trypsin), about 100 (etanercept) to about 2-8 (trypsin), about 100 (etanercept) to about 3-7 (trypsin), about 100 (etanercept) to about 4-6 (trypsin), about 100 (etanercept) to about 1-6 (trypsin), about 100 (etanercept) to about 3-9 (trypsin), about 100 (etanercept) to about 4.5-5.5 (trypsin), or about 100 (etanercept) to about 5 (trypsin) (i.e., about 20:1 etanercept:trypsin). In various aspects, the ratio of etanercept to Arg-C is about 100 (etanercept) to about 1-50 (Arg-C), such as about 100 (etanercept) to about 5-45 (Arg-C), about 100 (etanercept) to about 10-40 (Arg-C), about 100 (etanercept) to about 10-30 (Arg-C), about 100 (etanercept) to about 15-30 (Arg-C), about 100 (etanercept) to about 15-25 (Arg-C), about 100 (etanercept) to about 18-22 (Arg-C), about 100 (etanercept) to about 19-21 (Arg-C), about 100 (etanercept) to about 19.5-20.5 (Arg-C), or about 100 (etanercept) to about 20 (Arg-C) (i.e., about 20:4 etanercept:Arg-C). In various aspects of the disclosure, step (a) comprises digesting etanercept with trypsin and Arg-C at an etanercept:trypsin:Arg-C ratio of 20:1:4. The ratios are, in various aspects, weight ratios. The enzymes may be added to the digest reaction together. In this regard, the disclosure contemplates providing trypsin and Arg-C simultaneously in step (a) (i.e., the enzymes are provided near in time, such as within about five minutes of each other). Alternatively, the enzymes may be provided sequentially in step (a), such that an initial digest with trypsin is performed followed by addition of Arg-C to the reaction mix, or an initial digest with Arg-C is performed followed by addition of trypsin to the reaction mix.

Optionally, step (a) comprises digesting etanercept with trypsin and Arg-C and Lys-C to produce a pool of peptide fragments. Lys-C is an endoproteinase that cleaves at the carboxyl side of the amino acid lysine. The amounts of etanercept, trypsin, Arg-C, and Lys-C in the digest result in a ratio of about 100 (etanercept) to about 1 to about 100 (trypsin) to about 1 to about 100 (Arg-C) to about 1 to about 100 (Lys-C). In various aspects, the ratio of etanercept to Lys-C is about 100 (etanercept) to about 1-10 (Lys-C), such as about 100 (etanercept) to about 1-9 (Lys-C), about 100 (etanercept) to about 2-8 (Lys-C), about 100 (etanercept) to about 3-7 (Lys-C), about 100 (etanercept) to about 4-6 (Lys-C), about 100 (etanercept) to about 1-6 (Lys-C), about 100 (etanercept) to about 3-9 (Lys-C), about 100 (etanercept) to about 4.5-5.5 (Lys-C), or about 100 (etanercept) to about 5 (Lys-C) (i.e., about 20:1 etanercept: Lys-C).

The digest step (step (a)) may be performed under any suitable conditions which result in a digest suitable for examining glycosylation of etanercept, such as analysis methods using LC-MS. Optionally, step (a) is performed for about 1 to about 12 hours (e.g., about 1-10 hours, about 2-12 hours, about 1-9 hours, about 1-8 hours, about 1-7 hours, about 1-6 hours, about 1-5 hours, about 1-4 hours, about 1-3 hours, about 1.5-6 hours, about 2-8 hours, about 2-4 hours, or about 2-3 hours). In various aspects, step (a) is performed for about 2 hours. The digest step may be performed at any suitable temperature. In some embodiments, the digest step is performed at a temperature between about 20° C and about 45° C, or between about 20° C and about 40° C, or between about 22° C and about 40° C, or between about 25° C and about 37° C, such a temperature of about 37_{°} C ± 5_{°} C.

The digest step may be performed in the presence of a buffer. Suitable buffer components include, but are not limited to, tris(hydroxymethyl)aminomethane-HCl (Tris-HCl), CaCl₂, ethylenediaminetetraacetic acid (EDTA), and/or dithiothreitol (DTT). In various aspects of the disclosure, step (a) is performed in a buffer comprising Tris-HCI, CaCl₂, ethylenediaminetetraacetic acid (EDTA), and dithiothreitol (DTT). Optionally, about 25 mM to about 75 mM Tris-HCI is used, such as about 25 mM to about 50 mM Tris-HCI, about 50 mM to about 75 mM Tris-HCI, about 30 mM to about 60 mM Tris-HCI, about 45 mM to about 60 mM Tris-HCI, about 45 mM to about 55 mM Tris-HCI, or about 50 mM Tris-HCI. Optionally, about 1 mM to about 10 mM CaCl₂ is provided, such as about 1 mM to about 7.5 mM CaCl₂, about 3 mM to about 10 mM CaCl₂, about 3 mM to about 7.5 mM CaCl₂, about 4 mM to about 6 mM CaCl₂, or about 5 mM CaCl₂. Also optionally, about 0.5 mM to about 5 mM EDTA is provided, such as about 1 mM to about 4.5 mM EDTA, about 1 mM to about 3 mM EDTA, about 1.5 mM to about 3 mM EDTA, or about 2 mM EDTA. Optionally, about 0.5 mM to about 5 mM DTT is provided, such as about 1 mM to about 4.5 mM DTT, about 1 mM to about 3 mM DTT, about 1.5 mM to about 3 mM DTT, or about 2 mM DTT. For example, in a representative embodiment, step (a) is performed in the presence of about 50 mM Tris-HCI, about 5 mM CaCl₂, about 2 mM EDTA, and about 2 mM DTT. The pH of the digest is optionally about 7-8, such as about 7.8.

The digest results in a pool of peptide fragments. The peptide fragments are then applied to a reverse-phase chromatography column. Chromatographic methods separate peptide fragments in a mobile phase which is processed through a structure holding a stationary phase (e.g., a chromatography column). Because the polypeptide fragments are of different sizes and compositions, each fragment has its own partition coefficient. Because of the different partition coefficients, the polypeptides are differentially retained on the stationary phase. In reverse-phase columns, the stationary phase is a nonpolar hydrocarbon (e.g., silica derivatized with alkyl chains) and the mobile phase is a polar liquid. Reverse-phase columns include, e.g., hydrophilic interaction liquid chromatography (HILIC)-amide columns, HILIC-ZIC columns (e.g., zwitterionic stationary phase covalently attached to silica), C18 columns (chromatography column comprising octadecylsilane as stationary phase), C4 columns (chromatography column comprising butylsilane as stationary phase), C8 columns (chromatography column comprising octylsilane as stationary phase), Agilent AdvanceBio column, and phenyl-hexyl columns. Examples of chromatography columns for use in accordance with the methods described herein include columns comprising porous particles having a particle size of about 0.5 µm to about 7 µm (e.g., about 1 µm, about 2 µm, about 3 µm, about 4 µm, about 5 µm, about 6 µm, or about 7 µm, including ranges between any two of the listed values). The chromatography columns for use in the methods described herein may be at least 50 mm (e.g., at least 100 mm, at least 150 mm, at least 200 mm, at least 250 mm, or at least 300 mm) in height. In some aspects of the disclosure, the reverse-phase chromatography column used in the method is a C18 chromatography column, such as the Waters^{™} BEH (Ethylene Bridged Hybrid) Premier C18 column. Optionally, a C18 chromatography column is utilized which comprises tri-functionally bonded ethylene bridged hybrid particle with 1.7 µm particle size and wherein the column has an inner diameter of 2.1 mm and a length of 100 mm. Any suitable amount of peptide fragments can be applied to the chromatography column. In various instances, step (b) comprises injecting about 1 µg to about 25 µg of peptide fragments (e.g., about 5 µg to about 25 µg of peptide fragments) to the column.

The method further comprises eluting the peptide fragments from the chromatography column using a gradient consisting of variable amounts of a mobile phase A and a mobile phase B. Mobile phase A comprises (i) water and (ii) about 0.02%-0.5% formic acid or about 0.01%-0.5% trifluoroacetic acid (TFA). In various aspects, mobile phase A comprises water and formic acid. In this aspect, about 0.02%-0.5% formic acid is utilized (e.g., about 0.03%-0.45%, about 0.05%-0.35%, about 0.05%-0.25%, about 0.75%-0.25%, about 0.1%-0.25%, about 0.1%-0.5%, about 0.075%-0.25%, or about 1% formic acid). Alternatively or in addition, mobile phase A comprises water and about 0.01%-0.5% TFA (e.g., about 0.03%-0.45%, about 0.05%-0.35%, about 0.05%-0.25%, about 0.75%-0.25%, about 0.1%-0.25%, about 0.1%-0.5%, about 0.075%-0.25%, or about 1% TFA). Mobile phase B comprises (i) acetonitrile and (ii) about 0.02%-0.5% formic acid or about 0.01%-0.5% trifluoroacetic acid (TFA). In various aspects, mobile phase B comprises acetonitrile and formic acid. In this aspect, about 0.02%-0.5% formic acid is utilized in mobile phase B (e.g., about 0.03%-0.45%, about 0.05%-0.35%, about 0.05%-0.25%, about 0.75%-0.25%, about 0.1%-0.25%, about 0.1%-0.5%, about 0.075%-0.25%, or about 1% formic acid). Alternatively or in addition, mobile phase B comprises acetonitrile and about 0.01%-0.5% TFA (e.g., about 0.03%-0.45%, about 0.05%-0.35%, about 0.05%-0.25%, about 0.75%-0.25%, about 0.1%-0.25%, about 0.1%-0.5%, about 0.075%-0.25%, or about 1% TFA). In a representative embodiment, step (c) comprises eluting the peptide fragments using a gradient consisting of variable amounts of a mobile phase A comprising water and about 0.1% formic acid and a mobile phase B comprising acetonitrile and about 0.1% formic acid.

Step (c) utilizes any suitable flow rate to elute the peptides from the chromatography column. In various aspects, the flow rate is about 1 µL/min to about 800 µl/min, and the elution is performed over a time period of at least about 15 minutes. Optionally, the flow rate is at least about 50 µL/min, at least about 100 µL/min, at least about 150 µL/min, at least about 200 µL/min, at least about 250 µL/min, or at least about 300 µL/min, and no greater than about 800 µl/min, no greater than about 750 µl/min, no greater than about 700 µl/min, no greater than about 650 µl/min, no greater than about 600 µl/min, no greater than about 550 µl/min, or no greater than about 500 µl/min. In various aspects, flow rate is about 50 µL/min to about 750 µl/min, about 100 µL/min to about 750 µl/min, about 200 µL/min to about 750 µl/min, about 150 µL/min to about 500 µl/min, about 150 µL/min to about 600 µl/min, about 200 µL/min to about 600 µl/min, about 200 µL/min to about 400 µl/min, or about 200 µL/min to about 300 µl/min. In various aspects, the flow rate is about 250 µL/min. Step (c) is preferably performed over a time period of at least about 15 minutes (e.g., the exposure time of mobile phase B to the chromatography column is at least about 15 minutes). For example, the time period is optionally at least about 30 minutes, at least about 45 minutes, at least about 60 minutes, at least about 75 minutes, at least about 90 minutes, at least about 105 minutes, at least about 120 minutes, at least about 135 minutes, at least about 150 minutes, at least about 165 minutes, or at least about 180 minutes. Optionally, the time period is no longer than about 300 minutes, no longer than about 270 minutes, no longer than about 240 minutes, no longer than about 210 minutes, or no longer than about 180 minutes, although this is not required. In various aspects, step (c) is performed using a flow rate of about 200 µL/min to about 750 µl/min over a time period of at least about 120 minutes. The elution step may be performed under any suitable temperature, such as at a column temperature of about 50° C ± 5_{°} C.

In various aspects, the mobile phase gradient is performed under the following conditions:

| Time (min) | Mobile Phase A % | Mobile Phase B% |
|---|---|---|
| 0 | 99 | 1 |
| 5 | 99 | 1 |
| 12 | 90 | 10 |
| 100 | 69 | 31 |
| 101 | 69 | 31 |
| 106 | 30 | 70 |
| 111 | 30 | 70 |
| 112 | 10 | 90 |
| 116 | 10 | 90 |
| 117 | 99 | 1 |
| 127 | 99 | 1 |

In some aspects, the method described herein further comprises performing spectrometric analysis of the eluted peptide fragments. Exemplary methods for spectrometric analysis include, but are not limited to, mass spectrometry, ultraviolet spectrometry, visible light spectrometry, fluorescent spectrometry, ultraviolet-visible light spectrometry, and infrared spectrometry. In various aspects, the method comprises analyzing the eluted peptide fragments by mass spectrometric (MS) analysis. An illustrative MS instrument has three modules: an ion source, which converts gas phase sample molecules into ions (or, in the case of electrospray ionization, moves ions that exist in solution into the gas phase); a mass analyzer, which sorts the ions by their mass-to-charge ratios by applying electromagnetic fields; and a detector, which measures the value of an indicator quantity and thus provides data for calculating the abundances of each ion present. The principle underlying mass spectrometry (MS) includes ionizing chemical compounds to generate charged molecules or molecule fragments, and then measuring their mass-to-charge ratios. In an illustrative MS procedure, a sample is loaded onto the MS instrument and undergoes vaporization. The components of the sample are ionized by one of a variety of methods (e.g., by electrospray ionization), which results in the formation of positively charged particles. The positive ions are then accelerated by an electric field, and computations are performed on the mass-to-charge ratio (m/z) of the particles based on the details of motion of the ions as they transit through electric fields. The ions, which have been sorted according to their m/z ratios, are detected. Examples include gas chromatography-mass spectrometry (GC/MS or GC-MS); liquid chromatography mass spectrometry (LC/MS or LC-MS); ion mobility spectrometry/mass spectrometry (IMS/MS or IMMS); matrix-assisted laser desorption/ionization source configured with a TOF analyzer (MALDI-TOF); electrospray ionization-mass spectrometry (ESI-MS); inductively coupled plasma-mass spectrometry (ICP-MS); accelerator mass spectrometry (AMS); thermal ionization-mass spectrometry (TIMS); and spark source mass spectrometry (SSMS). In various aspects of the disclosure, LC-MS is performed on the eluted peptide fragments.

The Example below illustrates representative features of the disclosure. From the description of these aspects, other aspects of the invention can be made and/or practiced based on the description provided below. The methods involve use of molecular biological techniques described in treatises such as Molecular Cloning: A Laboratory Manual, 2nd ed., vol. 1-3, Sambrook et al., ed., Cold Spring Harbor Laboratory Press, Cold Spring Harbor, N.Y., 2001; and Current Protocols in Molecular Biology, Ausubel et al., ed., Greene Publishing and Wiley-Interscience, New York. The Example serves only to illustrate the invention and is not intended to limit the scope of the invention in any way.

### EXAMPLE

This Example describes the digest and evaluation of etanercept using the materials and methods described herein.

Etanercept is a heavily glycosylated protein. The 15th tryptic peptide (T15) is especially heavily sialylated. It was previously believed that identification of T15 glycopeptides using LC-MS required removal of sialic acid residues using, e.g., sialidase. Surprising, the method described herein enables identification of, e.g., T15 glycopeptides, without sialidase treatment, thereby preserving information about the biomolecule useful in evaluating stability.

Studies were conducted using various enzymes to digest etanercept: human neutrophil elastase (HNE), trypsin, Glu-C, proline specific endopeptidase, pepsin, chymotrypsin, pronase, proalanase, Lys-C, and Arg-C. In some instances, the combinations of enzymes were employed, either initially added to the reaction mix together (trypsin+Arg-C, Lys-C+Arg-C, or trypsin+Lys-C+Arg-C) or applied in sequential order. A representative buffer formulation included 50 mM tris(hydroxymethyl)aminomethane hydrochloride (Tris-HCL), pH 7.8, 5 mM CaCl₂, 2 mM ethylenediaminetetraacetic acid (EDTA) + fresh 2 mM DL-Dithiothreitol (DTT). Digestion time was about two hours.

Additionally, studies were conducted utilizing nine different HPLC columns: Hydrophilic interaction liquid chromatography (HILIC) Amide (functional group: amide), C18 (functional group: Octadecyl chain; Silica particle based Si-O-Si(CH3)2(CH2)17CH3), Premier C18 (C18 column having hybrid organic/inorganic surface technology that forms barrier surface between sample and column), porous graphitic carbon (PGC), ZIC^{®} HILIC, C4 (functional group: butyl chain; Silica particle based Si-O-Si(CH3)2(CH2)3CH3), C8 (functional group: octyl chain; Silica particle based Si-O-Si(CH3)2(CH2)7CH3), PhenylHexyl, and Agilent AdvanceBio (functional group: 120 angstrom poroshell particle C18). Premier C18 column provided the best separation for the etanercept peptide digest with gradient durations from ranging from 98 min to 186 min.

In order to quantify various sialyated species of the T15 peptide, the sample load on the liquid chromatography (LC) column was increased to 25 µg, rather than 5 µg which is commonly used for peptide mapping analysis. Six different T15 glycopeptides (each having the core amino acid sequence of SEQ ID NO: 1) were identified directly without sialidase treatment. See Table 1.

**TABLE 1**

| T15 Glycopeptide |
|---|
| SQHTQPTPEPSTAPSTSFLLPMGPSPPAEGSTGDEPK [2xHexNAcHexNeuAc] |
| SQHTQPTPEPSTAPSTSFLLPMGPSPPAEGSTGDEPK [3xHexNAcHexNeuAc] |
| SQHTQPTPEPSTAPSTSFLLPMGPSPPAEGSTGDEPK [4xHexNAcHexNeuAc] |
| SQHTQPTPEPSTAPSTSFLLPMGPSPPAEGSTGDEPK [5xHexNAcHexNeuAc] |
| SQHTQPTPEPSTAPSTSFLLPMGPSPPAEGSTGDEPK [6xHexNAcHexNeuAc] |

All three N-linked glycosylation and all the O-glycosylation species of etanercept were identified and quantified at the peptide level using the method described herein. Sialylation of etanercept reference standard was quantified using methods disclosed herein with a result of 20 moles per mole of etanercept and compared to the result of 15 moles per mole of etanercept from conventional sialic acid release and labeling HPLC method. The discrepancy could potentially be caused by incomplete release and labeling of sialic acid using conventional method and the superior quantification using methods disclosed herein. The instant methodology allows amino acid site-specific N-glycosylation identification and quantification, which has not previously been possible.

The peptide coverage resulting from digests using various enzymes and enzyme combinations is illustrated in Figures 1A-1I. A digest using trypsin and Arg-C was unexpectedly superior to others tested in terms of providing more complete coverage of etanercept.

While this invention has been described with an emphasis upon preferred embodiments, it will be obvious to those of ordinary skill in the art that variations of the preferred compounds and methods may be used and that it is intended that the invention may be practiced otherwise than as specifically described herein.

## Claims

1. A method of processing etanercept, the method comprising
(a) digesting etanercept with trypsin and Arg-C at an etanercept:trypsin:Arg-C ratio of 100:[1-100]:[1-100] to produce a pool of peptide fragments;
(b) applying the peptide fragments to a reverse-phase chromatography column; and
(c) eluting the peptide fragments using a gradient consisting of variable amounts of a mobile phase A comprising water and 0.02%-0.5% formic acid or 0.01%-0.5% trifluoroacetic acid (TFA) and a mobile phase B comprising acetonitrile and 0.02%-0.5% formic acid or 0.01%-0.5% TFA at a flow rate of 1 µL/min to 800 µl/min over a time period of at least 15 minutes.

2. The method of claim 1, wherein step (a) comprises digesting etanercept with trypsin and Arg-C at an etanercept:trypsin:Arg-C ratio of 20:1:4.

3. The method of claim 1 or claim 2, wherein the ratio is a weight ratio.

4. The method of any one of claims 1-3, wherein step (a) is performed for 1-12 hours at a temperature of 37_{°} C ± 5_{°} C.

5. The method of claim 4, wherein step (a) is performed for 2 hours.

6. The method of any one of claims 1-5, where step (a) is performed in a buffer comprising Tris-HCI, CaCl₂, ethylenediaminetetraacetic acid (EDTA), and dithiothreitol (DTT).

7. The method of any one of claims 1-6, wherein Arg-C and trypsin are provided simultaneously in step (a).

8. The method of any one of claims 1-6, wherein Arg-C and trypsin are provided sequentially in step (a).

9. The method of any one of claims 1-8, wherein the reverse-phase column is a C18 chromatography column.

10. The method of any one of claims 1-9, wherein step (b) comprises injecting 1 µg to 25 µg of peptide fragments to the column.

11. The method of any one of claims 1-10, wherein step (c) comprises eluting the peptide fragments using a gradient consisting of variable amounts of a mobile phase A comprising water and 0.1% formic acid and a mobile phase B comprising acetonitrile and 0.1% formic acid.

12. The method of any one of claims 1-11, wherein step (c) is performed using a flow rate of 200 µL/min to 750 µl/min over a time period of at least 120 minutes.

13. The method of any one of claims 1-12, wherein the flow rate of step (c) is 250 µl/min.

14. The method of any one of claims 1-13, wherein step (c) is performed at a column temperature of 50° C ± 5° C.

15. The method of any one of claims 1-14, further comprising (d) analyzing the eluted peptide fragments by spectrometric analysis.

## Patentansprüche

1. Verfahren zum Verarbeiten von Etanercept, wobei das Verfahren umfasst
(a) Verdauen von Etanercept mit Trypsin und Arg-C in einem Verhältnis von Etanercept : Trypsin : Arg-C von 100 : [1-100] : [1-100], um einen Pool von Peptidfragmenten zu erzeugen;
(b) Aufbringen der Peptidfragmente auf eine Umkehrphasen-Chromatographiesäule; und
(c) Eluieren der Peptidfragmente unter Verwendung eines Gradienten, bestehend aus variablen Mengen einer mobilen Phase A, die Wasser umfasst und 0,02 %-0,5 % Ameisensäure oder 0,01 %-0,5 % Trifluoressigsäure (TFA) umfasst, und einer mobilen Phase B, die Acetonitril und 0,02 %-0,5 % Ameisensäure oder 0,01 %-0,5 % TFA umfasst, bei einer Durchflussrate von 1 µl/min bis 800 µl/min über einen Zeitraum von wenigstens 15 Minuten.

2. Verfahren nach Anspruch 1, wobei Schritt (a) das Verdauen von Etanercept mit Trypsin und Arg-C in einem Verhältnis von Etanercept : Trypsin : Arg-C von 20 : 1 : 4 umfasst.

3. Verfahren nach Anspruch 1 oder 2, wobei das Verhältnis ein Gewichtsverhältnis ist.

4. Verfahren nach einem der Ansprüche 1-3, wobei Schritt (a) 1-12 Stunden lang bei einer Temperatur von 37 °C ± 5 °C durchgeführt wird.

5. Verfahren nach Anspruch 4, wobei Schritt (a) 2 Stunden lang durchgeführt wird.

6. Verfahren nach einem der Ansprüche 1-5, wobei Schritt (a) in einem Puffer durchgeführt wird, der Tris-HCl, CaCl₂, Ethylendiamintetraessigsäure (EDTA) und Dithiothreitol (DTT) umfasst.

7. Verfahren nach einem der Ansprüche 1-6, wobei Arg-C und Trypsin gleichzeitig in Schritt (a) bereitgestellt werden.

8. Verfahren nach einem der Ansprüche 1-6, wobei Arg-C und Trypsin nacheinander in Schritt (a) bereitgestellt werden.

9. Verfahren nach einem der Ansprüche 1-8, wobei die Umkehrphasensäule eine C18-Chromatographiesäule ist.

10. Verfahren nach einem der Ansprüche 1-9, wobei Schritt (b) das Injizieren von 1 µg bis 25 µg Peptidfragmenten in die Säule umfasst.

11. Verfahren nach einem der Ansprüche 1-10, wobei Schritt (c) das Eluieren der Peptidfragmente unter Verwendung eines Gradienten umfasst, der aus variablen Mengen einer mobilen Phase A, die Wasser und 0,1 % Ameisensäure umfasst, und einer mobilen Phase B besteht, die Acetonitril und 0,1 % Ameisensäure umfasst.

12. Verfahren nach einem der Ansprüche 1-11, wobei Schritt (c) unter Verwendung einer Durchflussrate von 200 µl/min bis 750 µl/min über einen Zeitraum von wenigstens 120 Minuten durchgeführt wird.

13. Verfahren nach einem der Ansprüche 1-12, wobei die Durchflussrate in Schritt (c) 250 µl/min beträgt.

14. Verfahren nach einem der Ansprüche 1-13, wobei Schritt (c) bei einer Säulentemperatur von 50 °C ± 5 °C durchgeführt wird.

15. Verfahren nach einem der Ansprüche 1-14, das ferner (d) das Analysieren der eluierten Peptidfragmente durch spektrometrische Analyse umfasst.

## Revendications

1. Procédé de traitement d'étanercept, le procédé comprenant :
(a) la digestion d'étanercept avec de la trypsine et Arg-C selon un rapport étanercept:trypsine:Arg-C de 100:[1 à 100]:[1 à 100] pour produire un groupe de fragments peptidiques ;
(b) l'application des fragments peptidiques à une colonne de chromatographie en phase inverse ; et
(c) l'élution des fragments peptidiques à l'aide d'un gradient constitué de quantités variables d'une phase mobile A comprenant de l'eau et de l'acide formique à 0,02 % à 0,5 % ou de l'acide trifluoroacétique (TFA) à 0,01 % à 0,5 % et d'une phase mobile B comprenant de l'acétonitrile et de l'acide formique à 0,02 % à 0,5 % ou du TFA à 0,01 % à 0,5 % à un débit de 1 µl/min à 800 µl/min sur une période d'au moins 15 minutes.

2. Procédé selon la revendication 1, dans lequel l'étape (a) comprend la digestion d'étanercept avec de la trypsine et Arg-C selon un rapport étanercept:trypsine:Arg-C de 20:1:4.

3. Procédé selon la revendication 1 ou la revendication 2, dans lequel le rapport est un rapport en poids.

4. Procédé selon l'une quelconque des revendications 1 à 3, dans lequel l'étape (a) est réalisée pendant 1 à 12 heures à une température de 37 °C ± 5 °C.

5. Procédé selon la revendication 4, dans lequel l'étape (a) est réalisée pendant 2 heures.

6. Procédé selon l'une quelconque des revendications 1 à 5, où l'étape (a) est réalisée dans un tampon comprenant Tris-HCl, CaCl₂, de l'acide éthylènediaminetétraacétique (EDTA), et du dithiothréitol (DTT).

7. Procédé selon l'une quelconque des revendications 1 à 6, dans lequel Arg-C et la trypsine sont fournis simultanément à l'étape (a).

8. Procédé selon l'une quelconque des revendications 1 à 6, dans lequel Arg-C et la trypsine sont fournis séquentiellement à l'étape (a).

9. Procédé selon l'une quelconque des revendications 1 à 8, dans lequel la colonne de phase inverse est une colonne de chromatographie C18.

10. Procédé selon l'une quelconque des revendications 1 à 9, dans lequel l'étape (b) comprend l'injection de 1 µg à 25 µg de fragments peptidiques dans la colonne.

11. Procédé selon l'une quelconque des revendications 1 à 10, dans lequel l'étape (c) comprend l'élution des fragments peptidiques à l'aide d'un gradient constitué de quantités variables d'une phase mobile A comprenant de l'eau et de l'acide formique à 0,1 % et d'une phase mobile B comprenant de l'acétonitrile et de l'acide formique à 0,1 %.

12. Procédé selon l'une quelconque des revendications 1 à 11, dans lequel l'étape (c) est réalisée à l'aide d'un débit de 200 µl/min à 750 µl/min sur une période d'au moins 120 minutes.

13. Procédé selon l'une quelconque des revendications 1 à 12, dans lequel le débit de l'étape (c) est de 250 µl/min.

14. Procédé selon l'une quelconque des revendications 1 à 13, dans lequel l'étape (c) est réalisée à une température de colonne de 50 °C ± 5 °C.

15. Procédé selon l'une quelconque des revendications 1 à 14, comprenant en outre (d) l'analyse des fragments peptidiques élués par analyse spectrométrique.
